# EUROPEAN PATENT APPLICATION

(11) **EP 3 815 607 A1**
(43) Date of publication of application: **05.05.2021**
(21) Application number: 19205635.6
(22) Date of filing: 28.10.2019
(51) Int. Cl.: A61B 5/11, A61B 5/00

(54) **DEVICE AND METHOD FOR MEASURING AND EVALUATING THE CURVATURE OF AT LEAST ONE BODY PART**

(71) Applicant: Razavidinani, Keyanoush, 50674 Köln (DE); Fischer, Roland, 52074 Aachen (DE)
(72) Inventor: RAZAVIDINANI, Keyanoush, 50674 Köln (DE); FISCHER, Roland, 52074 Aachen (DE)
(74) Representative: Jostarndt Patentanwalts-AG

(57) **Abstract**

The present invention refers to a sensor system (10) for measuring and evaluating the curvature of at least one body part of an individual, exhibiting at least one sensor device (11) with at least one flex sensor (11a), wherein the at least one flex sensor is embedded in silicone, wherein the at least one sensor device comprises an IMU (14), a microcontroller (15), and a battery (16), wherein the at least one sensor device (11) exhibits an adhesion interface (13), wherein the sensor system (10) further comprises a transmitting unit (17), an evaluating unit (18) and a feedback module (21), wherein the sensor system (10) is configured for evaluating the curvature based on measuring data acquired by the at least one flex sensor (11a), and wherein the sensor system (10) is further configured for evaluating the position and/or orientation of the body part. The present invention refers to a respective method

## Description

### TECHNCAL FIELD

The present invention relates to a sensor system for measuring and evaluating the curvature of movable and static bodies of individuals. In particular, the present invention relates to a flexible and stretchable sensor system configured for being applied to the spinal column. In particular, the present invention relates to methods and devices according to features of the claims.

### BACKGROUND

Many individuals have to look ahead in view of physical stress, or at least should take precautions. Learning new courses of movement, e.g. exercise executions in or outside the gym, or in context with correction of posture during pregnancy, working time or everyday life, may take at least 66 days or 450 repetitions until the movement becomes an automatism. Missing feedback about the movements may lead to wrong execution of the movements, which could harm the body and/or could even lead to pain. In case of permanently wrong execution, this wrong execution will be stored as a pattern instead of the correct one. In the long term, this can even lead to complaints in the musculoskeletal system. To avoid such long-term effects as well as short-term effects, a device for measuring posture in real-time is highly recommended and in great demand.

When the patients go to a physiotherapist, the specialist is only able to see the current state of the patients back. This problem creates the need for a non-invasive, easy-to-apply device that is able to monitor the patient's posture, especially the spinal column, and also the patient's movements over a longtime horizon.

In prior art, there are already devices configured for measuring the orientation of the upper back, especially by means of a single orientation sensor. These devices may encourage a healthier lifestyle, especially by encouraging an individual to bring the upper back in an upright position. They also may provide vibrational feedback.
Further products like those described in DE 10 2008 052 406 A1 or WO2016/058032 A1 or US 2010/0305482 A1 may even measure the bending and orientation of the whole back, but, these products do not seem to be quite practicable in use, especially due to their size and due to quite circumstantial attachment to the body.
Further, prior art comprises fitness training devices providing feedback by indirectly measuring exercise execution by means of a sensor attached to a barbell. But, likewise, these devices do not provide any information about the current curvature, orientation or position of the spine or the human body.
None of the devices introduced above may offer a sustainable and easy-to-apply system which allows for measuring the curvature, position, orientation, and acceleration of the spine.
Thus, there is a need for a consumer-friendly device which may provide for previously mentioned desirable characteristics.

### SUMMARY

It is an object of the present invention to provide for a device and method for facilitating monitoring of motion and/or posture of individuals.
It is also an object of the present invention to provide for a system allowing for great autarky while monitoring motion and/or posture of an individual.
It is also an object of the present invention to provide for a compact system allowing for robust and reliable data acquisition by any arbitrary individual, for evaluating motion and posture of the individual's spinal column.

At least one of these objects is solved by a sensor system configured for measuring and evaluating the curvature of at least one body part of an individual, especially of the body's spinal column, exhibiting at least one sensor device with at least one flex sensor, wherein the at least one flex sensor is embedded in silicone, wherein the at least one sensor device comprises an inertial measurement unit IMU, a microcontroller, and a battery, wherein the at least one sensor device exhibits an adhesion interface and is configured for adhering to the body part, especially for laminar two-dimensional adhesion over a length in the range of 2-30cm, preferably between 5 and 15 cm, wherein the sensor system further comprises a transmitting unit and an evaluating unit, wherein the sensor system is configured for evaluating the curvature based on measuring data acquired by the at least one flex sensor, and wherein the sensor system is further configured for evaluating the position and/or orientation of the body part, especially by evaluating data acquired by the IMU, especially by evaluating data acquired by the sensor device being arranged at the body part. This allows for reliable data acquisition, e.g. in an arrangement at the back, e.g. the lower back. The longitudinal axis of the sensor device can be arranged in alignment with the spinal column. Such a sensor system also provides for a great degree of autarky, both in view of energy consumption and data acquisition and evaluation. In particular, the sensor system may function without any external energy sources, and also without any further sensing devices (i.e., exclusively based on measuring data acquired in the position at the body part, e.g.at the spine, e.g. at the spine).

The invention captures the posture and movement execution of a user by means of analysing the curvature of the spine in combination with the position of the user relative to earths gravitational axis.

The present invention allows for acquisition and evaluation of data about the individual's daily posture, especially about the manner the back and especially the spinal column are behaving throughout the day. The data may e.g. be used by specialists in order to give a better diagnose about the reasons for a patient's (back) pain. In particular, the present invention may provide for improved feedback-loop between supervisors and patients and may also enable the patient to get more information about their own posture. The data may be provided in raw format and/or may be visualized on an application or software. The present invention allows for increasing the user's body awareness and for preventing bad posture and for reducing occurrence of pain.

Embedded in silicone and designed for being flexible and stretchable, the sensor device may easily align with the body's dynamic form and does not impede its movement. Additionally, it can be coupled with other positional and/or orientational tracking devices, e.g. those of fitness trackers, smart watches or smartphones.

Those previous mentioned tracking devices can be used to improve the measurement accuracy of the users posture and provide better feedback based on the movement of the arms if coupled with such a tracking device.

The flex sensor is configured for measuring an amount of deflection and/or bending in at least one spatial direction, especially in at least two spatial directions, preferably in all three spatial directions. In other words: The flex sensor may comprise several flex elements each providing for measurement data in at least one spatial direction. The flex sensor may be designated as unidimensional or multidimensional goniometer or flexible potentiometer also. Additionally, the flex sensor is either integrated into the sensor system or can be attached to it via a clip system.

The microcontroller (MCU) may exhibit RAM for memorizing tolerance data, especially predefined tolerance data which is specific for a defined individual (e.g. a human being at the age of 25 years), which allows for data matching, especially for matching with measured data. Such a configuration of the MCU also favors stand-alone functionality of the sensor device.

The at least one sensor device may exhibit at least one status LED and/or a power button, especially respectively encapsulated in silicone.

Silicones (polydimethylsiloxanes) have to be found to be preferred materials for embedment resp. encapsulation of the sensor system. Based on this technical teaching, the skilled person may find further materials having same or comparable material characteristics.

The IMU may comprise an accelerometer, a gyroscope, and optionally also a magnetometer, especially a 3-axis-accelerometer, a 3-axis-gyroscope, and optionally also a 3-axis-magnetometer.

Preferably, the sensor system is configured for calculating attitude and heading information. Based on data acquired by the IMU, velocity and distance travelled by the individual can be determined.

Further, it has been found that the whole sensor device may be reusable, especially since sole interface to the individual's body may be provided by the adhesion interface.

It has been found that for some applications, it is favorable to provide at least two flex sensors connected in parallel. In particular, it has to be found to be advantageous providing several flex sensors each acquiring measuring data only along a longitudinal section of the whole length of the sensor device. This can improve accuracy, especially in an arrangement at the back in alignment with the spinal column.

According to one embodiment the at least one sensor device is configured for adhering the body part by reversible adhesion, wherein the adhesion interface is double-sided adhesive. This also facilitates sustainable use and provides for easy handling and for individual optimization of adhesion properties for each individual body part.

It has to be noted that each flex sensor can be coupled with at least one strain gauge. In other words: The sensor device may also comprise strain gauges, especially as many strain gauges as flex sensor. The skilled person may also interchange/substitute a flex sensor by a strain gauge, when appropriate (especially depending on the application and also depending on the individual).

According to one embodiment the at least one sensor device is configured for measuring the bidirectional curvature and/or bending of the at least one body part, especially of the body's spinal column, especially in an arrangement solely at the spine (one single component system, one single device).

According to one embodiment the battery is configured (dimensioned/designed) for at least 24h of self-sufficient energetic functioning of the sensor device. This also provides for favorable energetic autarky.

According to one embodiment the sensor device is configured for stand-alone functionality (one single component system, one single device, especially in an arrangement at the spine), especially based on tolerance data memorized in the microcontroller. This may further improve autarky.

The measuring data may relate to curvature and/or bending in at least two spatial directions. In particular, "curvature" has to be understood as a flexion in the sagittal plane (i.e., back and forth), and "bending" has to be understood as a flexion in the frontal plane (i.e., left and right).

Stand-alone functionality allows for using the sensor device alone, without any further device. This also provides for good handling characteristics and good practicability.

The battery may ensure energetic autarky for at least 24h of standard functioning. It has been found that the most part of energy is needed for ensuring functioning of vibration modules (feedback modules). For stand-alone applications, the sensor device is run in low-power mode, especially with reduced data rate. In particular, data can be stored in an internal memory of the MCU. Also, interrupt events may be implemented which allow for triggering predefined data packages or data rates acquired by the sensors, wherein the data packages can be correlated to specific patterns of motion.

Where in the context of this patent application modular implementations of the invention are described, implementations with single component system, or single device can be used as well and vice versa.

According to one embodiment the at least one sensor device is designed to comprise at least two separate parts which can be docked/coupled to one another, namely a first part having relatively small dimensions (especially maximum 20-30% of the length of the second part) and/or at least approximately round or oval geometry, and a second part having relatively big dimensions (especially minimum 70-80% of the length of the whole sensor device) and/or at least approximately linear or bar-shaped or elongate flat geometry. This modularity also favors broad applicability, especially for different body parts.

According to one embodiment the at least one sensor device has a symmetric shape or composition with respect to at least one spatial direction. This may also facilitate correct application and alignment.

According to one embodiment, the sensor device has a length in the range of 2 to 30cm, especially of 5 to 15 cm. According to one embodiment the sensor device has a width in the range of 1 to 5cm, especially of 1 to 3cm. According to one embodiment the sensor device has an elongated design, especially with a length to width ration greater than 5 or even greater than 10. This respectively allows for favorable sensing characteristics especially for spinal column applications. By the way: The whole sensor device or even the whole sensor system can be accommodated in a housing resp. encapsulation not exceeding this spatial extension.

According to one embodiment the sensor device's length is non-adjustable or non-cuttable. This may also ensure correct use by any arbitral individual, resp. correct data acquisition.

According to one embodiment the at least one sensor device is designed to comprise at least two separate parts which can be docked/coupled to one another, namely a first part provided for sensing/measuring absolute and/or relative position, orientation and/or acceleration as well as evaluating and a second part provided for sensing/measuring curvature/bending. This configuration is also favorable in view of modularity resp. variability. The first and second parts may both be embedded/encapsulated in silicone.

According to one embodiment the at least one sensor device is structured in a first part at least comprising one of the IMU, the microcontroller, and the battery, and in a second part at least comprising the at least one flex sensor embedded in silicone. This modular concept allows for adapting the sensor characteristics and at the same time, conserving the composition of all further electronic components.

According to one embodiment the at least one sensor device exhibits a coupling interface configured for coupling the at least one flex sensor to at least one of: the IMU, the microcontroller, and the battery. This further facilitates realization of a modular concept.

According to one embodiment the at least one sensor device is configured for adhering the body part by reversible adhesion, wherein the adhesion interface is double-sided adhesive. This also facilitates sustainable use and provides for easy handling and for individual optimization of adhesion properties for each individual body part.

According to one embodiment the adhesion interface exhibits at least one adhesive tape or layer and/or at least one skin-friendly silicone adhesive. Silicone adhesive may also provide the advantage of being easily removable, e.g. by pulling it off, shower gel, body lotions or the like. Taping as means for applying e.g. physio tape to the body, is already a well-known method by physiotherapists to reduce bad posture or pain after it occurs. In context with the present invention, a flexible and stretchable adhesive has been found to be quite favorable, especially in view of alignment of flex sensors with, e.g., the spinal column.

According to one embodiment the at least one sensor device is configured for being attachable (resp. adhered) by one single hand to the respective body part at the adhesion interface. This further enhances straightforward use by any individual, even without any assistance.

According to one embodiment the adhesion interface exhibits at least one tape, especially a double-sided adhering tape, providing for characteristics of a kinesic tape and/or physio (physio-therapeutic) tape. Such tapes also provide for high stretchability and high tear strength.

According to one embodiment the at least one sensor device is stretchable by at least 50%, the at least one flex sensor being relatively movable with respect to the silicone embedment. This also allows for measuring quite considerable bending and curvature.

According to one embodiment the at least one sensor device, especially at least one inlay accommodating the at least one flex sensor, exhibits a stretchability of at least 50%, wherein the relative position of the flex sensor is defined either by a connection to a further sensor component within the silicone embedment or by punctual connection with the inlay. This may ensure a favorable relative position of the respective flex sensor.

Stretchability may e.g. also be ensured by embedding the flex sensor in an elastomer as PDMS or like that within the silicone encapsulation, which can be realized with or without provision of an inlay.

According to one embodiment the at least one flex sensor is fully encapsulated in silicone. According to one embodiment the IMU, the microcontroller, and the battery are fully encapsulated in silicone, and optionally also the transmitting unit, the evaluation unit, a feedback module, especially vibrational module, and a memory. According to one embodiment the at least one sensor device with the at least one flex sensor is fully encapsulated in silicone in all three spatial directions, especially in hermetic manner such that no fluid may get in contact with the sensor device. Full encapsulation provides for a compact closed sensor system and may seal off all the sensor components from the environment. In other words: The entire device, or even the whole system, can be arranged within one (hermetic) cover or casing/housing.

According to one embodiment the at least one sensor device exhibits a silicone embedment accommodating the at least one flex sensor, which allows for relative motion of the at least one flex sensor at least in longitudinal direction. This may ensure mechanical decoupling between the embedment/encapsulation and the flex sensor.

According to one embodiment the at least one flex sensor is embedded in an inlay, wherein the inlay is arranged within the silicone embedment of the sensor device. The inlay is configured for accommodating at least one flex sensor, preferably at least two or three flex sensors. The inlay may provide for a kind of cover or envelope around the flex sensor, especially in such a manner that the flex sensor may move relatively within the inlay. The inlay may exhibit material characteristics, especially flexibility, same as (or at least comparable with) those of silicone. In particular, the inlay is stretchable by at least 50% (i.e. up to 150% of its original dimensions). The inlay itself can be fully encapsulated from the outside, especially by silicone encapsulation. On its inner surfaces, the inlay may exhibit a coating providing for a gliding surface (a kind of sliding bearing) for the flex sensor(s). At the end facing away from the sensor components (especially at the remote end of a/the second sensor part), the inlay is closed such that no silicone may enter the cavity encased by the inlay. Especially due to the silicone's viscosity, even at the other end (nearby end next to sensor components), i.e., at the inlay's open end, no silicone may enter the quite narrow gap or slot. Flex sensors which are not directly connected (or which are not adjacent) to a circuit board may be connected punctually with the inlay and may be connected via flex cables to a circuit board resp. to the further sensor components. This configuration further favors stretching characteristics of the sensor device, without any risk of harming electronic components accommodated within the embedment/encapsulation. For example, the inlay may be provided as a kind of tube.

According to one embodiment the at least one sensor device, especially the at least one flex sensor, comprises at least one strain gauge. According to one embodiment the sensor system is configured for measuring at least one of the following parameters in conjunction with the curvature parameter: absolute and/or relative position, orientation and/or acceleration of the at least one body part. This respectively allows for a broader data basis.

According to one embodiment the at least one sensor device comprises a vibration module (feedback module). A vibration module (feedback module) may allow for haptic feedback, i.e., for prompt and silent interaction between the system and the individual. Preferably, at least one vibration module is integrated in the sensor device at a location nearby the IMU and/or the battery.

According to one embodiment the sensor system communicates with or comprises at least one smart watch and/or at least one fitness tracker, respectively exhibiting at least one IMU and respectively being coupled with the sensor device. This may also provide for a broader data basis. In contrast, prior art does not provide for integration of external sensors attached to other body parts in order to provide a more complete feedback system.

According to one embodiment the sensor system comprises at least one database and/or data cloud system. This may also favor data comparison and evaluation.

The present invention may also be described as follows:
The flexible and stretchable sensor system resp. sensor device can be fully encapsulated and embedded in silicone. Its length can be between 10 to 20 cm and its width between 1.0 to 3cm even more preferred 1.5 to 3cm. The sensor system may consist of at least one flex sensor and/or strain gauge, at least one inertial measurement unit (IMU), especially an IMU being provided as a combination of accelerometer, gyroscope, and optionally also magnetometer), a microcontroller (MCU), a transmitting unit, a feedback system, and a battery resp. accumulator. The flex sensor measures the bidirectional curvature and/or bending of a body part, e.g. of the back. The IMU detects the position and/or orientation of the body part it is attached to, as well as its relative position in space as well as forward, backward, and sideways bending. The MCU processes all measured sensor data from the flex sensor and the IMU. The sensor system can be used as a stand-alone system and/or may communicate (especially via bluetooth or other wireless or wired transmitters) with one or more computing devices (like a PC or
smartphone) to transmit acquired data. The feedback system (e.g. a vibration module or a
screen on the computing device) provides the user with (haptic, visual and/or audible) feedback about current curvature and posture of the body part. The sensor system is powered by a rechargeable battery cell and can be charged by wire and/or wirelessly.

The sensor device may align with the body part to which it is attached to because of its flexibility and stretchability. It can easily be attached with one hand to the body and has a non-adjustable resp. non-cuttable length of e.g. 5 to 15 cm. The connection of the sensor system to the body can be realized via an interchangeable adhesive tape (like a stretchable physio tape) and/or a skin-friendly silicone adhesive. This adhesive layer (adhesion interface) can easily be replaced, especially in case adhesion properties are getting worse, such that the sensor device is reusable.

Optionally, additional devices that include at least one IMU (like smart watches, fitness tracker, ...), attached to different body parts (e.g. wrist or ankle) can be added to the system, in order to acquire further data relating to relative movements between different points of the body, especially in order to create a 3D like image. For example, the system comprises at least one sensor device (resp. hand sensor) being attached to an individual's hand or being hold by an individual's hand.

Additionally, the acquired data can be uploaded to a database (and/or cloud system) and can be used to constantly improve motion detection. By feeding data of specific exercises, movements and postures into the database, specific exercises, movements, and postures can be detected, especially in order to compare the individual's current execution, movement and posture with optimal execution sets stored within the database, and for providing feedback for improving posture and body-awareness. Such optimal execution data sets can be stored in various ways: by the user/individual himself, by a professional trainer and/or a physiotherapist. It is recommended to use data sets that are verified by professionals and/or specialists having the necessary expertise for defining appropriate exercises, movements and/or posture executions.

The percentage of aberration between the current movement and a stored optimal execution data set can be set into an app, for example as maximal tolerance values of the simple feedback system. If the current movement exceeds the maximal tolerance value, the feedback system may give live and direct feedback to the individual (especially in form of optical feedback, vibration and/or acoustic feedback). The feedback system enables users to compare different sets of the same movements, postures and/or exercises, and to show/illustrate recorded data to professional trainers and/or physiotherapists, and also to see/analyze their movements in a more secure and sustainable way, especially in order to avoid bad posture. The feedback system can also be used to inform the wearer about that they should change their position when resting, sitting or like that too long in the same position.

At least one of the above mentioned objects is also solved by a method of measuring and evaluating the curvature of at least one body part of an individual, especially of the body's spinal column, especially at the spine, by means of a sensor system comprising at least one sensor device with at least one flex sensor embedded in silicone, the sensor device adhering to the body part, the method comprising acquisition of measuring data by the at least one flex sensor being arranged at the body part as well as evaluation of the curvature based on acquired measuring data and further evaluation of the position and/or orientation of the body part based on data acquired by an inertial measurement unit IMU of the sensor device, especially by evaluating data acquired by the IMU being arranged at the body part. This provides for reliable and robust data acquisition and evaluation by means of a compact system, which may optionally be used as a single-component system, i.e., exclusively in an arrangement at the respective body part.

According to one embodiment, based on measuring data acquired by the at least one flex sensor, the position and/or orientation of the at least one body part is detected by the IMU and evaluated in such a manner with respect to time that the individual's motion is deductible by the sensor system. This also allows for correlation of posture data with motion data, in order to more reliably evaluate the individual's movement pattern.

According to one embodiment movement execution data characterizing the individual's motion is acquired by at least one further IMU arranged at a further body part.
According to one embodiment movement patterns are deduced from evaluated measuring data and are correlated with predefined movement patterns, or the movement execution data is correlated with predefined movement patterns or individual movement patterns of specific individuals.
According to one embodiment a feedback, especially a vibrational feedback, is provided to the individual, in case a/the detected movement corresponds to a specific movement pattern.

These method steps respectively allow for more detailed analysis and interaction. Further, data from each individual may be evaluated, e.g. body height, age, weight, gender, etc. The feedback can be provided as haptic, acoustic and/or visual feedback. The patterns of motion can be deduced based on data measured by acceleration sensors, gyroscopes, flex sensors, and optionally further sensors.

Based on roll and pitch angles, especially detected by the respective IMU, the system may calibrate the sensor device even in case the flex sensors are not in correct alignment with the respective body part.

At least one of the above mentioned objects is also solved by a sensor system for measuring and evaluating the curvature of at least one body part of an individual, especially of the body's spinal column, especially at the spine, especially sensor system according to any of the preceding device claims, processed or fabricated by providing at least one sensor device with at least one flex sensor for acquiring measuring data and with an inertial measurement unit IMU for evaluating the position and/or orientation of the body part, with a microcontroller, and with a battery, and by embedding or encapsulating at least the at least one flex sensor in silicone, especially by also embedding or encapsulating the IMU, the microcontroller, and the battery in silicone, and by further providing an adhesion interface configured for adhering the sensor device to the body part. This provides for above mentioned advantages.

The invention can also be summarized as follows.
A fully encapsulated and reusable flexible and stretchable sensor system embedded in silicone comprising at least one flex sensor, inertial measurement unit IMU, microcontroller, transmitting unit and optionally also a feedback system provides for improved data acquisition and evaluation, especially with respect to an individual's posture of the spinal column throughout the day or the night. The sensor system is e.g. 5-15 cm long and 1-3cm wide and can be attached, for example, to the back of the human body. In this way, the spinal curvature, position, and/or orientation can be determined by means of a compact and autarkic system which is easy to apply by any individual. In particular, the sensor system can easily be attached one-handed to the body, especially by means of an interchangeable adhesive tape and/or a skin-friendly silicone adhesive (adhesion interface). This adhesive layer can easily be replaced if adhesion properties are getting worse, so that the sensor system is reusable. Additional devices comprising at least one inertial measurement unit IMU (e.g. smart watches or fitness trackers with integrated IMU) can also be coupled with the sensor system or integrated therein. All sensor data can temporarily be stored in an internal memory of the sensor system and/or, if a connection to a computing device (smartphone, laptop,...) is active, the data can be transmitted to one or more of them. For long-term storage, all data can be uploaded to a database. The recorded data represents movement executions. The recorded data can also be evaluated by comparing it with predefined patterns of motion.

### BRIEF DESCRIPTION OF FIGURES

The present invention is exemplarily illustrated by referring to the following figures. The figures respectively show:
**Figure 1** in a perspective view an encapsulated sensor device provided for a sensor system according to one embodiment;
**Figure 2** in a perspective view sensor elements and flex sensors of a sensor device provided for a sensor system according to one embodiment;
**Figure 3** in schematic illustration a sensor system according to a further embodiment;
**Figure 4** in a perspective view a sensor system resp. sensor device according to a further embodiment; and
**Figure 5** in a sectional view a sensor system resp. sensor device according to one of the embodiments.

### DETAILED DESCRIPTION OF THE FIGURES

To begin with, reference signs are described generally en masse. Specific reference is made to some of the relevant reference signs in context with each figure.

The present invention provides for a sensor system 10 comprising at least one sensor device 11 exhibiting at least one flex sensor 11a, wherein the flex sensor 11a provides for measuring data relating to the orientation resp. posture of an individual's body part, especially of the spine. An embedment 12 resp. encapsulation 12, especially silicone embedment, provides for a housing for sensor components.

According to one embodiment, the sensor device 11 exhibits at least two parts, namely a first part 11.1 and a second part 11.2, which parts can be connected via a female connector 11.3 and a male connector 11.4, especially by means of a coupling resp. coupling interface 11.5 in the form of a clip connection.

According to one embodiment, the at least one flex sensor 11a is accommodated within an inlay 12.1 encased by the embedment 12 resp. encapsulation 12.

An adhesion interface (body interface) 13 provides for adhesion to the body part. The adhesion interface may comprise, e.g., double-sided adhesive tape.

The sensor device 11 further comprises an inertial measurement unit (IMU) 14, a microcontroller (MCU) 15 with internal RAM, and a battery resp. accumulator 16. Further, a transmitting unit 17 and an evaluating unit 18 may be provided as further components of the sensor device or the sensor system. A power button 19 and a status LED 20 and a feedback module 21, especially vibration module, allow for expedient and convenient interaction with the user resp. individual. Acquired measuring data can be memorized in memory 22.

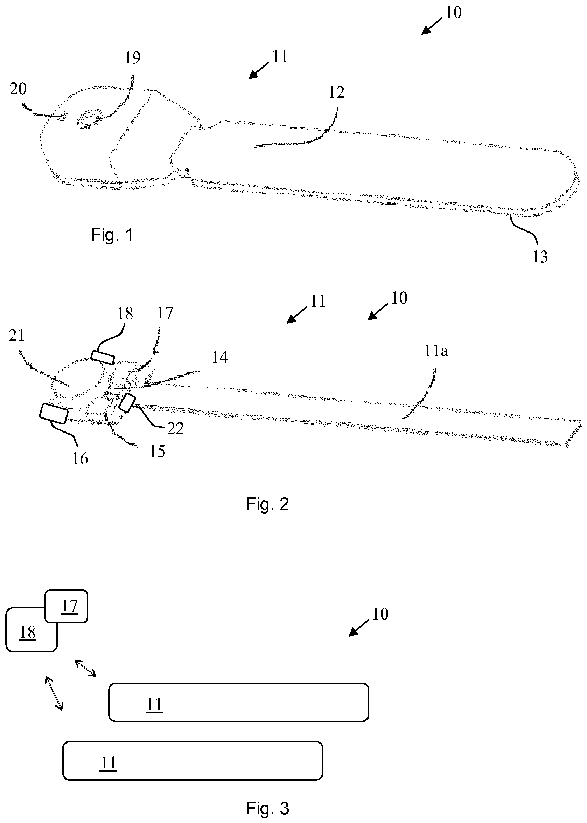

Fig. 1 illustrates a fully encapsulated sensor device 11, especially in a stand-alone configuration (autarky while being in used attached to the respective body part).

Fig. 2 shows an arrangement of several sensor elements of the sensor device according to the invention. It can be seen that the flex sensor 11a extends along a section in which no further electronic sensor components are provided. In other words: A first sensing cluster is provided by all electronic components exclusive the flex sensor, and a second sensing cluster is provided by the flex sensor(s) resp. by sensors acquiring data referring to the curvature of the body part.

In Fig. 2, there is shown a single flex sensor 11a. Nonetheless, the sensor device 11 may comprise several flex sensors 11a.

Fig. 3 illustrates a sensor system 10 exhibiting several sensor devices 11 and an additional evaluating unit 18 which may be provided by a smart watch for example, or by fitness trackers being worn at the wrist or ankle. Such a system may allow for more complex analysis and/or real-time evaluation by further users.

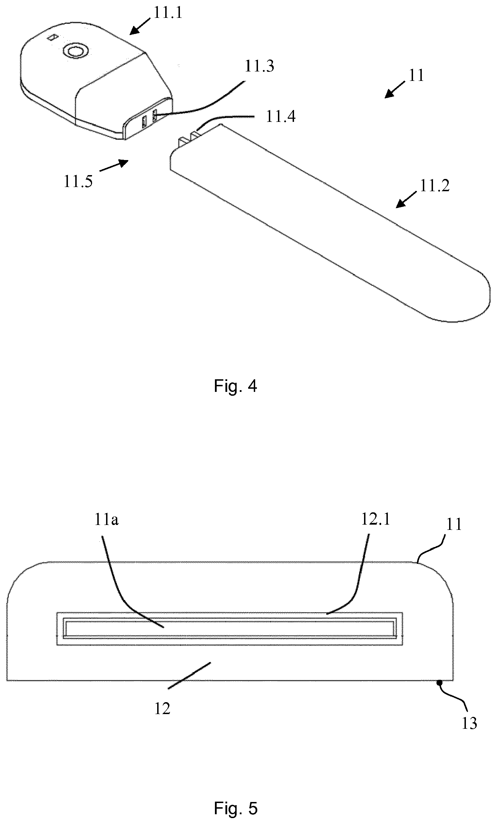

Fig. 4 shows a modular sensor concept, the sensor device 11 exhibiting two parts 11.1, 11.2 being connectable via coupling interface 11.5. Each part 11.1, 11.2 can be embedded in silicone, and the coupling interface may provide for at least approximately hermetic sealing of electronic connectors and the like. The arrangement of the sensor components may for example be realized as described in context with Fig. 2.

Fig. 5 illustrates a cross section through the elongated part of the sensor device. The at least one flex sensor 11a is arranged within in an inlay 12.1, and the inlay is encapsulated by silicone.

It is apparent, that the invention is not limited to one sensor, but that more sensors, especially two or three sensors could be used as well.

In some advantageous embodiments at least two sensors partially overlap.

In preferred embodiments of the invention sensors could be arranged in a plane.

In advantageous embodiments of the invention sensors could be arranged in a plane separated from each other.

### List of reference signs

- 10: sensor system
- 11: sensor device
- 11.1: first part of sensor device
- 11.2: second part of sensor device
- 11.3: female connector, especially female clip connection
- 11.4: male connector, especially male clip connection
- 11.5: coupling interface
- 11a: flex sensor
- 12: embedment resp. encapsulation, especially silicone embedment
- 12.1: inlay
- 13: adhesion interface (body interface)
- 14: inertial measurement unit (IMU)
- 15: microcontroller (MCU) with internal RAM
- 16: battery resp. accumulator
- 17: transmitting unit
- 18: evaluating unit
- 19: power button
- 20: status LED
- 21: feedback module, especially vibration module
- 22: memory

## Claims

1. Sensor system (10) configured for measuring and evaluating the curvature of at least one body part of an individual, especially of the body's spinal column, especially at the spine, exhibiting at least one sensor device (11) with at least one flex sensor (11a), wherein the at least one flex sensor is embedded in silicone, wherein the at least one sensor device comprises an inertial measurement unit IMU (14), a microcontroller (15), and a battery (16),
wherein the at least one sensor device (11) exhibits an adhesion interface (13) and is configured for adhering to the body part, especially for laminar two-dimensional adhesion over a length in the range of 5-15cm,
wherein the sensor system (10) further comprises a transmitting unit (17) and an evaluating unit (18),
wherein the sensor system (10) is configured for evaluating the curvature based on measuring data acquired by the at least one flex sensor (11a),
and wherein the sensor system (10) is further configured for evaluating the position and/or orientation of the body part, especially by evaluating data acquired by the IMU (14), especially by evaluating data acquired by the sensor device being arranged at the body part.

2. Sensor system according to claim 1, wherein the at least one sensor device is configured for measuring the bidirectional curvature and/or bending of the at least one body part, especially of the body's spinal column, especially in an arrangement solely at the lower spine; and/or wherein the battery is configured for at least 24h of self-sufficient energetic functioning of the sensor device; and/or wherein the sensor device is configured for stand-alone functionality, especially based on tolerance data memorized in the microcontroller.

3. Sensor system according to any of the preceding claims, wherein the at least one sensor device is designed to comprise at least two separate parts which can be docked/coupled to one another, namely a first part having relatively small dimensions and/or at least approximately round or oval geometry, and a second part having relatively big dimensions and/or at least approximately linear or bar-shaped or elongate flat geometry; and/or wherein the at least one sensor device has a symmetric shape or composition with respect to at least one spatial direction; and/or wherein the sensor device has a length in the range of 5 to 30cm, especially of 10 to 20cm; and/or wherein the sensor device has a width in the range of 1 to 5cm, especially of 1 to 3cm; and/or wherein the sensor device has an elongated design, especially with a length to width ration greater than 5 or even greater than 10; and/or wherein the sensor device's length is non-adjustable or non-cuttable.

4. Sensor system according to any of the preceding claims, wherein the at least one sensor device is designed to comprise at least two separate parts which can be docked/coupled to one another, namely a first part provided for sensing/measuring and a second part provided for evaluating; and/or wherein the at least one sensor device is structured in a first part at least comprising one of the IMU, the microcontroller, and the battery, and in a second part at least comprising the at least one flex sensor embedded in silicone; and/or wherein the at least one sensor device exhibits a coupling interface configured for coupling the at least one flex sensor to at least one of: the IMU, the microcontroller, and the battery.

5. Sensor system according to any of the preceding claims, wherein the at least one sensor device is configured for adhering the body part by reversible adhesion, wherein the adhesion interface is double-sided adhesive; and/or wherein the adhesion interface exhibits at least one adhesive tape or layer and/or at least one skin-friendly silicone adhesive; and/or wherein the at least one sensor device is configured for being attachable by one single hand to the respective body part at the adhesion interface; and/or wherein the adhesion interface exhibits at least one tape, especially a double-sided adhering tape, providing for characteristics of a kinesic tape and/or physio tape.

6. Sensor system according to any of the preceding claims, wherein the at least one sensor device is stretchable by at least 50%, the at least one flex sensor being relatively movable with respect to the silicone embedment; and/or wherein the at least one sensor device, especially at least one inlay accommodating the at least one flex sensor, exhibits a stretchability of at least 50%, wherein the relative position of the flex sensor is defined either by a connection to a further sensor component within the silicone embedment or by punctual connnection with the inlay.

7. Sensor system according to any of the preceding claims, wherein the at least one flex sensor is fully encapsulated in silicone; and/or wherein the IMU, the microcontroller, and the battery are fully encapsulated in silicone, and optionally also the transmitting unit, the evaluation unit, a feedback module, especially vibrational module, and a memory; and/or wherein the at least one sensor device with the at least one flex sensor is fully encapsulated in silicone in all three spatial directions, especially in hermetic manner such that no fluid may get in contact with the sensor device; and/or wherein the at least one sensor device exhibits a silicone embedment accommodating the at least one flex sensor, which allows for relative motion of the at least one flex sensor at least in longitudinal direction; and/or wherein the at least one flex sensor is embedded in an inlay, wherein the inlay is arranged within the silicone embedment of the sensor device.

8. Sensor system according to any of the preceding claims, wherein the at least one sensor device, especially the at least one flex sensor, which can comprise one strain gauge; and/or wherein the sensor system is configured for measuring at least one of the following parameters in conjunction with the curvature parameter: absolute and/or relative position, orientation and/or acceleration of the at least one body part; and/or wherein the at least one sensor device comprises a vibration module.

9. Sensor system according to any of the preceding claims, wherein the sensor system communicates with or comprises at least one smart watch and/or at least one fitness tracker, respectively exhibiting at least one IMU and respectively being coupled with the sensor device; and/or wherein the sensor system comprises at least one database and/or data cloud system.

10. Method of measuring and evaluating the curvature of at least one body part of an individual, especially of the body's spinal column, , by means of a sensor system (10) comprising at least one sensor device (11) with at least one flex sensor (11a) embedded in silicone, the sensor device (11) adhering to the body part, the method comprising acquisition of measuring data by the at least one flex sensor (11a) being arranged at the body part as well as evaluation of the curvature based on acquired measuring data and further evaluation of the position and/or orientation of the body part based on data acquired by an inertial measurement unit IMU (14) of the sensor device (11), especially by evaluating data acquired by the IMU (14) being arranged at the body part.

11. Method according to the preceding method claim, wherein based on measuring data acquired by the at least one flex sensor, the position and/or orientation of the at least one body part is detected by the IMU and evaluated in such a manner with respect to time that the individual's motion is deductible by the sensor system.

12. Method according to any of the preceding method claims, wherein movement execution data characterizing the individual's motion is acquired by at least one further IMU arranged at a further body part; and/or wherein movement patterns are deduced from evaluated measuring data and are correlated with predefined movement patterns, or the movement execution data is correlated with predefined movement patterns or individual movement patterns of specific individuals; and/or wherein a feedback, especially a vibrational feedback, is provided to the individual, in case a/the detected movement corresponds to a specific movement pattern.

13. Sensor system (10) for measuring and evaluating the curvature of at least one body part of an individual, especially of the body's spinal column, especially at the spine, especially sensor system according to any of the preceding device claims, processed or fabricated by providing at least one sensor device (11) with at least one flex sensor (11a) for acquiring measuring data and with an inertial measurement unit IMU (14) for evaluating the position and/or orientation of the body part, with a microcontroller (15), and with a battery (16), and by embedding or encapsulating at least the at least one flex sensor (11a) in silicone, especially by also embedding or encapsulating the IMU, the microcontroller, and the battery in silicone, and by further providing an adhesion interface (13) configured for adhering the sensor device to the body part.
